# NEW EUROPEAN PATENT SPECIFICATION

(11) **EP 2 329 848 B2**
(45) Date of publication and mention of the opposition decision: **19.06.2019**
(45) Mention of the grant of the patent: 24.10.2012
(21) Application number: 09175877.1
(22) Date of filing: 13.11.2009
(51) Int. Cl.: A61K 31/155, A61K 38/26, A61K 38/28, A61K 45/06

(54) **Lixisenatide as add-on therapy to insulin glargine and metformin for treating type 2 diabetes**
Lixisenatide zusätzlich zu Metformin und Insulin-glargin bei der Behandlung von Typ 2-Diabetes
Lixisénatide en tant que traitement d'appoint à l'insuline glargine et à la metformine dans le diabète de type 2

(43) Date of publication of application: 08.06.2011
(73) Proprietor: Sanofi-Aventis Deutschland GmbH, 65929 Frankfurt am Main (DE)
(72) Inventor: Silvestre, Louise, 75013 Paris (FR); Souhami, Elisabeth, 75013 Paris (FR); Wei, Xiaodan, Bridgewater, NJ 08807 (US)
(74) Representative: Weickmann & Weickmann PartmbB

(56) References cited:
- ARNOLDS S; DELLWEG S; CLAIR J; DAIN M; NAUCK M A; RAVE K; KAPITZA C: "Insulin Glargine (GLAR) plus Metformin (MET): An Efficacious and Safe Regimen That Can Be Combined with Exenatide (EXE) or Sitagliptin (SITA)" DIABETES, vol. 58, no. Suppl. 1, June 2009 (2009-06) , page A141, XP009130958 69TH ANNUAL MEETING OF THE AMERICAN-DIABETES-ASSOCIATION; NEW ORLEANS, LA, USA; JUNE 05 -09, 2009 ISSN: 0012-1797
- TEWS D; WERNER U; ECKEL J: "Enhanced protection against cytokine- and fatty acid-induced apoptosis in pancreatic beta cells by combined treatment with glucagon-like peptide-1 receptor agonists and insulin analogues" HORMONE AND METABOLIC RESEARCH, vol. 40, no. 3, March 2008 (2008-03), pages 172-180, XP009130880 ISSN: 0018-5043
- YKI-JARVINEN H; KAUPPINEN-MAKELIN R; TIIKKAINEN M; VAHATALO M; VIRTAMO H; NIKKILA K ET AL.: "Insulin glargine or NPH combined with metformin in type 2 diabetes: the LANMET study" DIABETOLOGIA, vol. 49, no. 3, March 2006 (2006-03), pages 442-451, XP002573816 ISSN: 0012-186X
- CAMPAS C, CASTANER R: "AVE-0010 GLP-1 Receptor Agonist Treatment of Diabetes" DRUGS OF THE FUTURE,, vol. 33, no. 10, 1 October 2008 (2008-10-01), pages 838-840, XP002572655

### SEQUENCE LISTING

<110> Sanofi-Aventis Deutschland GmbH
<120> Method of treatment of diabetes type 2 comprising add-on therapy to insulin glargine and metformin
<130> DE2009/200
<160> 2
<170> PatentIn version 3.3
<210> 1
   <211> 44
   <212> PRT
   <213> Artificial
<220>
   <223> AVE0010 (44 AS)
   Position 44 (Lys) is amidated
<400> 1
   His Gly Glu Gly Thr Phe Thr Ser Asp Leu Ser Lys Gln Met Glu Glu
   1               5                   10                  15
   Glu Ala Val Arg Leu Phe Leu Glu Trp Leu Lys Asn Gly Gly Pro Ser
               20                  25                  30
   Ser Gly Ala Pro Pro Ser Lys Lys Lys Lys Lys Lys
           35                  40
<210> 2
   <211> 39
   <212> PRT
   <213> Artificial
<220>
   <223> Heloderma Suspectum
   Position 39 (Ser) is amidated
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35

## Description

Subject of the present invention is a combination for use in the treatment of diabetes mellitus type 2, the combination comprising
(a) desPro³⁶Exendin-4(1-39)-Lys₆-NH₂ or/and a pharmaceutically acceptable salt thereof,
(b) insulin glargine or/and a pharmaceutically acceptable salt thereof, and
(c) metformin or/and a pharmaceutically acceptable salt thereof.

Metformin is a biguanide hypoglycemic agent used in the treatment of Type 2 diabetes mellitus not responding to dietary modification. Metformin improves glycemic control by improving insulin sensitivity. Metformin is usually administered orally.

Insulin is a polypeptide having 51 amino acid residues. Insulin consists of the A chain having 21 amino acid residues, and the B chain having 30 amino acid residues. The chains are coupled by 2 disulfide bridges. Insulin formulations have been used for a long time for therapy of diabetes mellitus type 1 and 2. Recently, insulin derivatives and insulin analogues have been used.

However, control diabetes mellitus type 2 by metformin and insulin may be insufficient. Thus, in these patients, additional measures for controlling diabetes mellitus type 2 may be required.

A first aspect of the present invention is a combination for use in the treatment of diabetes mellitus type 2 comprising
(a) desPro³⁶Exendin-4(1-39)-Lys₆-NH₂ (AVE0010, lixisenatide) or/and a pharmaceutically acceptable salt thereof,
(b) insulin glargine or/and a pharmaceutically acceptable salt thereof, and
(c) metformin or/and a pharmaceutically acceptable salt thereof.

The compounds of (a), (b) and (c) may be administered to a subject in need thereof, in an amount sufficient to induce a therapeutic effect.

The compound des Pro³⁶Exendin-4(1-39)-Lys₆-NH₂ (AVE0010, lixisenatide) is a derivative of Exendin-4. AVE0010 is disclosed as SEQ ID NO:93 in WO 01/04156:
- SEQ ID NO: 1 AVE0010 (44 AS).
- SEQ ID NO: 2 Exendin-4 (39 AS)

Exendins are a group of peptides which can lower blood glucose concentration. The Exendin analogue AVE0010 is characterised by C-terminal truncation of the native Exendin-4 sequence. AVE0010 comprises six C-terminal lysine residues not present in Exendin-4.

In the context of the present invention, AVE0010 includes pharmaceutically acceptable salts thereof. The person skilled in the art knows pharmaceutically acceptable salts of AVE0010. A preferred pharmaceutically acceptable salt of AVE0010 employed in the present invention is acetate.

AVE0010 (desPro³⁶Exendin-4(1-39)-Lys₆-NH₂) or/and a pharmaceutically acceptable salt thereof may be administered parenterally, e.g. by subcutaneous injection. Suitable injection devices, for instance the so-called "pens" comprising a cartridge comprising the active ingredient, and an injection needle, are known. AVE0010 or/and a pharmaceutically acceptable salt thereof may be administered in a suitable amount, for instance in an amount in the range of 10 to 15 µg per dose or 15 to 20 µg per dose once a day (progressive titration from 10 to 15 and to 20 µg /day. 20 µg is the effective maintenance dose).

In the present invention, AVE0010 or/and a pharmaceutically acceptable salt thereof may be administered in a daily dose in the range of 10 to 15 µg or in the range of 15 to 20 µg (progressive titration from 10 to 15 and to 20 µg /day. 20 µg is the effective maintenance dose). AVE0010 or/and a pharmaceutically acceptable salt thereof may be administered by one injection per day.

Insulin glargine (Lantus) is Gly(A21)-Arg(B31)-Arg(B32)-human insulin. In the context of the present invention, insulin glargine includes pharmaceutically acceptable salts thereof.

Insulin glargine or/and a pharmaceutically acceptable salt thereof may be administered by injection (by subcutaneous injection). Suitable injection devices, for instance the so-called "pens" comprising a cartridge comprising the active ingredient, and an injection needle, are known. Insulin glargine or/and a pharmaceutically acceptable salt thereof may be administered in a suitable amount, for instance in an amount of at least 10 units per day (the initial dose is 10 units; 80 units is the maximal dose possible with the pen with 1 injection)

In the present invention, insulin glargine or/and a pharmaceutically acceptable salt thereof may be administered in a daily dose of at least 10 units. Insulin glargine or/and a pharmaceutically acceptable salt thereof may be administered by one injection per day.

In the present invention, AVE0010 or/and a pharmaceutically acceptable salt thereof may be provided in a liquid composition The skilled person knows liquid compositions of AVE0010 suitable for subcutaneous administration.

In the present invention, insulin glargine or/and a pharmaceutically acceptable salt thereof may be provided in a liquid composition The skilled person knows liquid compositions of insulin glargine suitable for subcutaneous administration.

A liquid composition employed herein may have an acidic or a physiologic pH. An acidic pH preferably is in the range of pH 1 - 6.8, pH 3.5 - 6.8, or pH 3.5 - 5. A physiologic pH preferably is in the range of pH 2.5 -8.5, pH 4.0 to 8.5, or pH 6.0 to 8.5. The pH may be adjusted by a pharmaceutically acceptable diluted acid (typically HCl) or pharmaceutically acceptable diluted base (typically NaOH).

The preferred pH is in the range of pH 3,5 to 5,0.

The liquid composition may contain a buffer, such as a phosphate, a citrate, an acetate. Preferably, it can contain an acetate buffer, in quantities up to 5 µg/mL, up to 4 µg/mL or up to 2 µg/mL.

The liquid composition employed herein may comprise a suitable preservative. A suitable preservative may be selected from phenol, m-cresol, benzyl alcohol and p-hydroxybenzoic acid ester. A preferred preservative is m-cresol. However, the preferred liquid composition does not contain a preservative.

The liquid composition employed herein may comprise a tonicity agent. A suitable tonicity agent may be selected from glycerol, lactose, sorbitol, mannitol, glucose, NaCl, calcium or magnesium containing compounds such as CaCl₂. The concentration of glycerol, lactose, sorbitol, mannitol and glucose may be in the range of 100 - 250 mM. The concentration of NaCl may be up to 150 mM. A preferred tonicity agent is glycerol.

In addition, the liquid composition may contain L-methionin from 0,5 µg/mL to 20 µg/mL, preferably from 1 µg/mL to 5 µg/mL. Preferably it contains L-methionin.

Metformin is the international nonproprietary name of 1,1-dimethylbiguanide (CAS Number 657-24-9). In the present invention, the term "metformin" includes any pharmaceutically acceptable salt thereof.

In the present invention, metformin may be administered orally. The skilled person knows formulations of metformin suitable for treatment of diabetes type 2 by oral administration. Metformin may be administered in a dose of at least 1.5 g/day. For oral administration, metformin may be formulated in a solid dosage form, such as a tablet or pill.

In the present invention, desPro³⁶Exendin-4(1-39)-Lys₆-NH₂ or/and a pharmaceutically acceptable salt is administered in an add-on therapy to administration of metformin and insulin glargine.

In the present invention, the terms "add-on", "add-on treatment" and "add-on therapy" relate to treatment of diabetes mellitus type 2 with metformin, insulin glargine and AVE0010. Metformin, insulin glargine and AVE0010 may be administered within a time interval of 24 h. Metformin, insulin glargine and AVE0010 each may be administered in a once-a-day-dosage. Metformin may be administered by a different administration route than insulin glargine and AVE0010. Metformin may be administered orally, whereas AVE0010 and insulin glargine may be administered subcutaneously.

The subject to be treated by the combination of the present invention may have a fasting plasma glucose concentration of at least 7 mmol/L or/and 2 hours postprandial plasma glucose of at least 11.1 mmol/L. The subject may have a HbA1c value in the range of 7% to 10%.

The subject to be treated by the combination of the present invention may be an adult subject. The subject may have an age in the range of 18 to 50 years.

The combination of the present invention preferably is a combination for use in the treatment of a subject suffering from diabetes type 2, wherein diabetes type 2 is not adequately controlled by treatment with metformin and insulin alone, for instance with a dose of at least 1.5 g/day metformin and a dose of insulin of at least 10 units, preferably of 15 to 80 U/day for 3 months.

Another aspect of the present invention is a pharmaceutical combination comprising
(a) des Pro³⁶Exend in-4(1-39)-Lys₆-NH₂ or/and a pharmaceutically acceptable salt thereof,
(b) insulin glargine or/and a pharmaceutically acceptable salt thereof, and
(c) metformin or/and a pharmaceutically acceptable salt thereof.

Preferably, the combination of the present invention is for treatment of diabetes mellitus type 2.

Yet another aspect of the present invention is the use of a combination comprising
(a) desPro³⁶Exendin-4(1-39)Lys₆-NH₂ or/and a pharmaceutically acceptable salt thereof,
(b) insulin glargine or/and a pharmaceutically acceptable salt thereof, and
(c) metformin or/and a pharmaceutically acceptable salt thereof,
for the production of a medicament for the treatment of diabetes mellitus type 2.

The medicament comprises desPro³⁶Exendin-4(1-39)-Lys₆-NH₂, insulin glargine and metformin in separate formulations, as described herein.

The invention is further illustrated by the following example.

### Example

### 24-week treatment of diabetes type 2 with lixisenatide (AVE0010) as add-on therapy to insulin glargine and metformin

Subject of the example is a randomized, placebo-controlled, 2-arm parallel-group, multicenter study with a 24-week double-blind treatment period assessing the efficacy and safety of Lixisenatide in patients with type 2 diabetes insufficiently controlled with insulin glargine and metformin.

### Study primary objectives

The primary objective of this study is to assess the effects on glycemic control of lixisenatide in comparison to placebo as an add-on treatment to insulin glargine and metformin over a period of 24 weeks.

### Study secondary objectives

The secondary objectives are :
• To assess the effects of lixisenatide (AVE0010) on the percentage of patients reaching HbA1c <7 % and < or = 6.5 %, on plasma glucose (fasting, postprandial during a standardized meal challenge test, 7-point self monitored profiles), body weight, insulin glargine doses.
• To evaluate lixisenatide safety and tolerability as add on treatment to insulin glargine and metformin.
• To assess the impact of lixisenatide on treatment satisfaction using the Diabetes Treatment Satisfaction Questionnaire (state) (DTSQs) in the participating countries where it is validated.

| Condition | Intervention | Phase |
|---|---|---|
| Type 2 Diabetes Mellitus | Drug: lixisenatide (AVE0010) | Phase III |
| | Drug: placebo | |
| | Drug: insulin glargine (HOE901) | |

| | |
|---|---|
| Study Type: | Interventional |
| Study Design: | Treatment, Randomized, Double Blind (Subject, Caregiver, Investigator, Outcomes Assessor), Placebo Control, Parallel Assignment, Efficacy Study |

### Primary Outcome Measures:

- Change in glycated hemoglobin (HbA1c) (time frame: 24 weeks, designated as safety issue: no)

### Secondary Outcome Measures:

- Percentage of patients with HbA1c <7 %, < or = 6.5 % (time frame: 24 weeks, designated as safety issue: no)
- Change in postprandial plasma glucose (time frame: 24 weeks, designated as safety issue: no)
- Change in fasting plasma glucose (time frame: 24 weeks, designated as safety issue: no)
- Change in 7-point Self Monitored Plasma Glucose (SMPG) profiles (time frame: 24 weeks, designated as safety issue: no)
- Change in body weight (time frame: 24 weeks, designated as safety issue: no)
- Change in insulin glargine dose (time frame: 24 weeks, designated as safety issue: no)
- Percentage of patients requiring rescue therapy during the double-blind period (time frame: 24 weeks, designated as safety issue: no)
- Change in treatment satisfaction score (DTSQ questionnaire, time frame: 24 weeks, designated as safety issue: no)

### Estimated Enrolment:

| Arms | Assigned interventions |
|---|---|
| Lixisenatide: Experimental 24-week treatment with lixisenatide once daily on top of insulin glargine (both injected in the morning within 1 hour prior to breakfast) and metformin (at least 1.5g/day) | Drug: lixisenatide (AVE0010) solution for subcutaneous injection |
| | Drug: insulin glargine (HOE901) solution for subcutaneous injection |
| Placebo: Placebo Comparator 24-week treatment with placebo once daily on top of insulin glargine (both injected in the morning within 1 hour prior to breakfast) and metformin (at least 1.5g/day) | Drug: placebo solution for subcutaneous injection |
| | Drug: insulin glargine (HOE901) solution for subcutaneous injection |

### Detailed Description

The study will comprise 3 periods:
- An up-to 14-week screening period, which includes an up to 2-week screening phase and a 12-week run-in phase with introduction and titration of insulin glargine on top of metformin +/-TZDs.
- At the end of the run-in phase, patients whose HbA1 c (centralized assay) is > or = 7% and < or = 9% and whose mean fasting SMPG calculated from the self measurements for the 7 days prior to visit 12 (week -1) is less than or equal to 126 mg/dl (7.0 mmol/l), will enter a 24-week double-blind randomized treatment period comparing lixisenatide to placebo (on top of insulin glargine + metformin +/-TZDs).
- A 3 day-safety follow up period.
Maximum duration of 39 weeks ± 7 days

### Eligibility

| | |
|---|---|
| Ages Eligible for Study: | 18 Years and older |
| Genders Eligible for Study: | Both |
| Accepts Healthy Volunteers: | No |

### Inclusion criteria:

At screening
- Patients with type 2 diabetes mellitus, as defined by WHO (fasting plasma glucose > or = 7 mmol/L (126mg/dL) or 2 hours postprandial plasma glucose > or = 11.1 mmol/L (200 mg/dL), diagnosed at least 1 year before the screening visit
- For at least 3 months: treatment with a stable dose of metformin > or = 1.5 g/day or combination of stable doses of metformin > or = 1.5 g/day with sulfonylureas (SUs) (to be stopped at visit 1) and/or Thiazolidinediones (TZDs)
- Glycated hemoglobin (HbA1c) > or = 7.0 and < or = 10%

At the end of the run in phase and before randomization:
- HbA1c> or = 7.0 and <or = 9%
- Mean fasting Self Monitored Plasma Glucose (SMPG) calculated from the self measurements for the 7 days prior to visit 12 (week -1) is less than or equal to 126 mg/dll (7.0 mmol/l)

### Exclusion criteria:

At screening:
- Pregnancy or lactation
- Women of childbearing potential with no effective contraceptive method.
- Type 1 diabetes mellitus
- Metformin not at a stable dose of at least 1.5 g/day for at least 3 months prior to the screening visit.
- Use of oral or injectable antidiabetic or hypoglycemic agents other than metformin, sulfonylurea and thiazolidinediones within 3 months prior to the time of screening, use of weight loss drugs if not at a stable dose for at least 3 months prior to the screening visit.
- History of hypoglycemia unawareness.
- History of unexplained pancreatitis, chronic pancreatitis, pancreatectomy, stomach/gastric surgery, inflammatory bowel disease
- History of metabolic acidosis, including diabetic ketoacidosis within 1 year prior to screening
- Hemoglobinopathy or hemolytic anemia, blood or plasma products transfusion within 3 months prior to the time of screening
- Within the last 6 months prior to screening: history of myocardial infarction, stroke, or heart failure requiring hospitalization
- Known history of drug or alcohol abuse within 6 months prior to the time of screening
- Uncontrolled or inadequately controlled hypertension at the time of screening with a resting systolic or diastolic blood pressure >180 mmHg or >110 mmHg, respectively
- Use of systemic glucocorticoids (excluding topical application or inhaled forms) for one week or more within 3 months prior to the time of screening
- Use of any investigational drug within 3 months prior to screening
- Renal impairment defined with serum creatinine >1.4 mg/dL in women and >1.5 mg/dL in men
- History of hypersensitivity to insulin glargine or to any of the excipients
- Clinically relevant history of gastrointestinal disease associated with prolonged nausea and vomiting, including (but not limited to): gastroparesis, unstable (i.e worsening) and not controlled (i.e prolonged nausea and vomiting) gastroesophageal reflux disease requiring medical treatment, within 6 months prior to the time of screening
- Any previous treatment with lixisenatide (e.g. participation in a previous study with lixisenatide)
- Allergic reaction to any GLP-1 receptor agonist in the past (e.g. exenatide, liraglutide) or to metacresol

Additional exclusion criteria during or at the end of the run-in phase before randomization:
- Informed consent withdrawal (patient who is not willing to continue or fails to return)
- Mean fasting SMPG calculated from the self-measurements for the 7 days prior to visit 12 (week -1) is >126 mg/dl (7.0 mmol/l)
- HbA1 c measured at visit 12 (week -1) is <7% or >9 %,
- Amylase and/or lipase >3 times the upper limit of the normal laboratory range at visit 12 (week -1)

The above information is not intended to contain all considerations relevant to a patient's potential participation in a clinical trial.

## Claims

1. A combination for use in the treatment of diabetes mellitus type 2, the combination comprising
(a) desPro³⁶Exendin-4(1-39)-Lys₆-NH₂ or/and a pharmaceutically acceptable salt thereof,
(b) insulin glargine or/and a pharmaceutically acceptable salt thereof, and
(c) metformin or/and a pharmaceutically acceptable salt thereof.

2. The combination for use according to claim 1, wherein desPro³⁶Exendin-4(1-39)-Lys₆-NH₂ or/and a pharmaceutically acceptable salt thereof is administered subcutaneously.

3. The combination for use according to claim 1 or 2, wherein insulin glargine or/and a pharmaceutically acceptable salt thereof is administered subcutaneously.

4. The combination for use according to any one of the claims 1 to 3, wherein the metformin is administered orally.

5. The combination for use according to any one of the preceding claims, wherein desPro³⁶Exendin-4(1-39)-Lys₆-NH₂ or/and a pharmaceutically acceptable salt is administered in an add-on therapy to administration of metformin and insulin glargine.

6. The combination for use according to any one of the preceding claims, wherein the subject to be treated is an adult subject.

7. The combination for use according to any one of the preceding claims, wherein diabetes mellitus type 2 is not adequately controlled with metformin and insulin alone.

8. The combination for use according to claim 7, wherein treatment with a dose of at least 1.5 g/day metformin and of at least 10 units/day of insulin alone for three months does not adequately control diabetes mellitus type 2

9. The combination for use according to anyone of the preceding claims, wherein the subject to be treated has a HbA1 c value in the range of 7% to 10% or/and a fasting plasma glucose concentration of at least 7 mmol/L or/and 2 hours postprandial plasma glucose of at least 11.1 mmol/L.

10. A pharmaceutical combination comprising
(a) desPro³⁶Exendin-4(1-39)-Lys₆-NH₂ or/and a pharmaceutically acceptable salt thereof,
(b) insulin glargine or/and a pharmaceutically acceptable salt thereof, and
(c) metformin or/and a pharmaceutically acceptable salt thereof.

11. The combination of claim 10, wherein the combination is for use in the treatment of diabetes mellitus type 2.

## Patentansprüche

1. Kombination zur Verwendung in der Behandlung von Diabetes mellitus Typ 2, wobei die Kombination umfasst
(a) desPro³⁶Exendin-4(1-39)-Lys₆-NH₂ oder/und ein pharmazeutisch akzeptables Salz davon,
(b) Insulin glargin oder/und ein pharmazeutisch akzeptables Salz davon, und
(c) Metformin oder/und ein pharmazeutisch akzeptables Salz davon.

2. Kombination zur Verwendung gemäß Anspruch 1, wobei desPro³⁶Exendin-4(1-39)-Lys₆-NH₂ oder/und ein pharmazeutisch akzeptables Salz davon subkutan verabreicht wird.

3. Kombination zur Verwendung gemäß Anspruch 1 oder 2, wobei Insulin glargin oder/und ein pharmazeutisch akzeptables Salz davon subkutan verabreicht wird.

4. Kombination zur Verwendung gemäß einem der Ansprüche 1 bis 3, wobei Metformin oral verabreicht wird.

5. Kombination zur Verwendung gemäß einem der vorhergehenden Ansprüche, wobei desPro³⁶Exendin-4(1-39)-Lys₆-NH₂ oder/und ein pharmazeutisch akzeptables Salz in einer Add-On-Therapie zur Verabreichung von Metformin und Insulin glargin verabreicht wird.

6. Kombination zur Verwendung gemäß einem der vorhergehenden Ansprüche, wobei die zu behandelnde Person eine erwachsene Person ist.

7. Kombination zur Verwendung gemäß einem der vorhergehenden Ansprüche, wobei Diabetes mellitus Typ 2 durch Metformin und Insulin allein nicht ausreichend behandelt wird.

8. Kombination zur Verwendung gemäß Anspruch 7, wobei die Behandlung mit einer Dosis von mindestens 1,5 g/Tag Metformin und mindestens 10 Einheiten/Tag Insulin allein für drei Monate den Diabetes mellitus Typ 2 nicht ausreichend behandelt.

9. Kombination zur Verwendung gemäß einem der vorhergehenden Ansprüche, wobei die zu behandelnde Person einen HbA1c-Wert im Bereich von 7% bis 10% oder/und eine Nüchternplasmaglukosekonzentration von mindestens 7 mmol/L oder/und eine 2 Stunden postprandiale Plasmaglukose von mindestens 11,1 mmol/L hat.

10. Pharmazeutische Kombination umfassend
(a) desPro³⁶Exendin-4(1-39)-Lys₆-NH₂ oder/und ein pharmazeutisch akzeptables Salz davon,
(b) Insulin glargin oder/und ein pharmazeutisch akzeptables Salz davon, und
(c) Metformin oder/und ein pharmazeutisch akzeptables Salz davon.

11. Kombination nach Anspruch 10, wobei die Kombination zur Verwendung in der Behandlung von Diabetes mellitus Typ 2 vorgesehen ist.

## Revendications

1. Combinaison pour l'utilisation dans le traitement du diabète sucré de type 2, la combinaison comprenant :
(a) la desPro³⁶Exendin-4(1-39)-Lys₆-NH₂ ou/et un sel pharmaceutiquement acceptable de celle-ci,
(b) l'insuline glargine ou/et un sel pharmaceutiquement acceptable de celle-ci, et
(c) la metformine ou/et un sel pharmaceutiquement acceptable de celle-ci.

2. Combinaison pour l'utilisation selon la revendication 1, dans laquelle la desPro³⁶Exendin-4(1-39)-Lys₆-NH₂ ou/et un sel pharmaceutiquement acceptable de celle-ci est administrée par voie sous-cutanée.

3. Combinaison pour l'utilisation selon la revendication 1 ou 2, dans laquelle l'insuline glargine ou/et un sel pharmaceutiquement acceptable de celle-ci est administrée par voie sous-cutanée.

4. Combinaison pour l'utilisation selon l'une quelconque des revendications 1 à 3, dans laquelle la metformine est administrée par voie orale.

5. Combinaison pour l'utilisation selon l'une quelconque des revendications précédentes, dans laquelle la desPro³⁶Exendin-4(1-39)-Lys₆-NH₂ ou/et un sel pharmaceutiquement acceptable de celle-ci est administrée en tant que traitement d'appoint à l'administration de metformine et d'insuline glargine.

6. Combinaison pour l'utilisation selon l'une quelconque des revendications précédentes, dans laquelle le sujet à traiter est un sujet adulte.

7. Combinaison pour l'utilisation selon l'une quelconque des revendications précédentes, dans laquelle le diabète sucré de type 2 n'est pas contrôlé de manière adéquate avec la metformine et l'insuline seules.

8. Combinaison pour l'utilisation selon la revendication 7, dans laquelle le traitement avec une dose d'au moins 1,5 g/jour de metformine et d'au moins 10 unités/jour d'insuline seules pendant trois mois ne contrôle pas de manière adéquate le diabète sucré de type 2.

9. Combinaison pour l'utilisation selon l'une quelconque des revendications précédentes, dans laquelle le sujet à traiter a une valeur d'HbA1c dans la plage de 7% à 10% ou/et une concentration de glucose plasmatique à jeun d'au moins 7 mmol/l ou/et de glucose plasmatique 2 heures après repas d'au moins 11,1 mmol/l.

10. Combinaison pharmaceutique comprenant
(a) la desPro³⁶Exendin-4(1-39)-Lys₆-NH₂ ou/et un sel pharmaceutiquement acceptable de celle-ci,
(b) l'insuline glargine ou/et un sel pharmaceutiquement acceptable de celle-ci, et
(c) la metformine ou/et un sel pharmaceutiquement acceptable de celle-ci.

11. Combinaison selon la revendication 10, dans laquelle la combinaison est destinée à l'utilisation dans le traitement du diabète sucré de type 2.
